# EUROPÄISCHE PATENTSCHRIFT

(11) **EP 0 683 391 B1**
(45) Veröffentlichungstag und Bekanntmachung des Hinweises auf die Patenterteilung: **09.12.1998**
(21) Anmeldenummer: 95104600.2
(22) Anmeldetag: 29.03.1995
(51) Int. Cl.: G01N 27/416

(54) **Messanordnung zur Untersuchung gasförmiger Medien**
Measuring arrangement for examining gaseous media
Dispositif de mesure pour l'examen de milieux gazeux

(30) Priorität: 20.05.1994 DE 4417665
(43) Veröffentlichungstag der Anmeldung: 22.11.1995
(73) Patentinhaber: TESTO GmbH & Co., D-79849 Lenzkirch (DE)
(72) Erfinder: Senn, Jürgen, D-79868 Freiburg (DE); Strnad, Kurt, D-79853 Lenzkirch (DE)
(74) Vertreter: Patentanwälte Westphal, Buchner, Mussgnug Neunert, Göhring

(56) Entgegenhaltungen:
- EP-A- 0 016 351
- DE-A- 4 214 599
- DE-C- 3 928 697
- DE-U- 9 105 996
- US-A- 5 331 310

## Beschreibung

Die Erfindung betrifft eine Meßanordnung zur Untersuchung gasförmiger Medien, die im wesentlichen aus den in Anspruch 1 mit Ziffer 1.1 bis 1.3 definierten Bauteilen besteht.

Meßanordnungen dieser Art weisen Meßzellen oder Meßsensoren, z. B. elektrochemische Gassensoren, auf, mit deren Hilfe gasförmige Medien, insbesondere toxische und nichttoxische Gase, untersucht werden. Üblicherweise werden die Meßzellen an den gasführenden Kanälen bzw. den Meßkammern mittels lösbarer Schraubverbindungen befestigt. Bei diesen Befestigungsarten, insbesondere bei der Befestigung mit über den Umfang der Meßzelle verteilter Schrauben, treten folgende Probleme auf, die deshalb besonders kritisch sind, weil die Meßzellen nach einer bestimmten Benutzungsdauer oder im Falle des Defektes ausgetauscht werden müssen.

Werden die Schrauben zur Befestigung der Meßzelle nicht gleichmäßig angezogen, so ergibt sich eine ungleichmäßige Flächenpressung, was zu Undichtheiten im Meßsystem führen kann. Da über die Undichtheiten Nebenluft in die Meßzelle gelangt, wird das Meßergebnis verfälscht.

Der ungleichmäßige Druck führt ferner zu Verspannungen des Meßzellen- bzw. Sensorgehäuses, welche Materialrisse zur Folge haben können. Durch diese Risse kann entweder wiederum Nebenluft in das Meßsystem gelangen oder es tritt Flüssigkeit aus dem Inneren der Meßzelle aus. Beide Defekte führen zu Meßwertverfälschungen und letztlich zur Unbrauchbarkeit der Meßzelle.

Diese Nachteile können nur vermieden werden, wenn die Befestigungsschrauben zur Einhaltung eines maximalen Drehmomentes mit einem Drehmomentenschlüssel angezogen werden, der dem Praktiker in der Regel nicht zur Verfügung steht. Außerdem muß bei einem Meßzellenaustausch darauf geachtet werden, daß die Befestigungsschrauben zur Erzielung eines gleichmäßigen Dichtungsdruckes "über Kreuz" angezogen werden.

Ein weiterer Nachteil ist, daß mechanische Krafteinwirkungen auf das Meßzellengehäuse bzw. auf die die Auswerteschaltung tragende Platine, soweit diese mit dem Meßzellengehäuse in mechanischer Verbindung steht, zu Meßwertverfälschungen führen. Das bedeutet aber, daß nach einem Meßzellenaustausch das Meßsystem erneut abgeglichen werden muß. Hierfür ist normalerweise ein Prüfgas erforderlich, welches aber dem Anwender häufig nicht zur Verfügung steht.

In der Praxis wird dieses Problem dadurch gelöst, daß die Meßzelle werkseitig ausgemessen und mit einem entsprechenden Koeffizienten gekennzeichnet wird, mit welchem der Anwender die Auswerteschaltung für die jeweilige Meßzelle nach erfolgtem Einbau ohne Verwendung von Prüfgas einstellen kann. Aber auch eine derartige Einstellung setzt gleiche und reproduzierbare Einbauverhältnisse der Meßzelle voraus, wie sie bei Bestimmung der Koeffizienten im Werk gegeben waren. Ist dies nicht gewährleistet, treten wiederum Meßfehler auf.

Will man diese Fehler vermeiden, sind zwei wichtige Randbedingungen zu beachten.

Zum einen muß die Anordnung so ausgebildet sein, daß die Meßzelle in reproduzierbarer Weise stets unter gleichen Bedingungen montierbar ist.

Zum anderen muß die Auswerte- und Verarbeitungselektronik so mit der Meßzelle verbunden sein, daß ein nachträglicher Abgleich beim Anwender nicht notwendig ist.

Es ist zwar bekannt, die Platinen mit der Signalauswerteschaltung zusammen mit der Meßzelle, mit welcher sie elektrisch verbunden ist, auszuliefern. Da jedoch eine sichere Verbindung zwischen Meßzelle und der Platine fehlt, können Erschütterungen beim Einsatz zu Kontaktproblemen und damit wiederum zu Meßfehlern führen. Das Verschrauben der Platine mit der Meßzelle ist problematisch, da mechanische Biegebeanspruchungen zu Rißbildungen und/oder Meßfehlern führen können.

So liegt der vorliegenden Erfindung die Aufgabe zugrunde, eine Meßanordnung zu schaffen, welche einen problemlosen Austausch der abgeglichenen Meßzelle mit der Signalauswerteschaltung ermöglicht, wobei die Anordnung derart sein soll, daß Montagefehler, die direkt oder indirekt zu Meßwertverfälschungen führen, weitgehend ausgeschlossen werden.

Gelöst wird diese Aufgabe mit einer Meßanordnung, wie sie im Anspruch 1 im einzelnen angegeben ist.

Die Grundidee der Erfindung besteht darin, die Meßzelle mit der die Auswerteschaltung tragenden Platine sicher zu verbinden und diese Einheit in einer Aufnahme zu montieren, welche gleichmäßige und reproduzierbare Druckbeanspruchungen der Meßzelle und Platine gewährleistet.

Bei dieser Art der Meßzellenbefestigung kann dem Anwender eine exakt abgeglichene Meßzelle geliefert werden, welche nach Austausch ohne nachträgliches Abgleichen oder Einstellen eingesetzt werden kann.

Die wesentlichen Elemente der Aufnahme bestehen hierbei aus einer Grundplatte und einer Druckplatte, zwischen welchen die Meßzelle mit der Platine eingespannt ist. Die Druckplatte, die mit der Grundplatte über Verbindungsarme lösbar verbunden ist, liegt unter der Wirkung einer Druckfeder mit definierter Federspannung gleichmäßig z. B. mit Druckstützen oder einem Druckring auf der Meßzelle auf. Hierbei können diese Druckelemente unmittelbar auf der Oberfläche der Meßzelle aufliegen, oder, wie ferner vorgeschlagen ist, in an der Meßzelle vorgesehene Kanäle oder Aufnahmen kraftschlüssig eingreifen.

Konstruktive Einzelheiten dieser Lösung sind mit den Unteransprüchen im einzelnen angegeben.

So besteht gemäß Anspruch 2 die Druckfeder zweckmäßigerweise aus einer Blattfeder, welche beidendig in die achsparallel verlaufenden Verbindungsarme eingehängt ist. Hierbei ist die Blattfeder an zwei Punkten gelagert und so ausgebildet, daß sie mit einer vorgegebenen Kraft über die Druckplatte die Meßzelle gegen die Grundplatte drückt. Die Blattfeder ist hierbei auf der einen Seite in einen Stehbolzen eingehängt und auf der anderen Seite mit einem hakenförmigen Ende eines an die Grundplatte schwenkbar angelenkten Armes verbunden. Eine derart gestaltete Halterung ermöglicht dem Anwender, durch Aushängen der Blattfeder die Meßzelle mit einem Handgriff ohne Verwendung von Werkzeugen in einfacher Weise auszutauschen.

Denkbar ist es hierbei auch, das Federelement selbst z. B. über ein Scharnier mit dem Stehbolzen bleibend zu verbinden. Auch kann die Anordnung von Druckplatte und/oder Stehbolzen durch ein einstückiges Gehäuseteil ersetzt werden. Ebenso denkbar sind andere Ausbildungen des Federelementes, nach welchen anstelle der hakenförmigen Verbindung eine Art Bajonettverschluß vorgesehen ist.

Gemäß Merkmal Ziffer 1.4 des Anspruchs 1 ist die die Signalverarbeitungsschaltung tragende Platine elektrisch und mechanisch direkt mit der Meßzelle verbunden. Hierdurch entfallen empfindliche Leitungsverbindungen, die insbesondere bei geringen Strömen und Spannungen oft zu Meßwertverfälschungen führen. Die Platine ist hierbei auch mechanisch gesichert, da sie zwischen der Meßzelle und der Druckplatte festgelegt ist. Auf ihr können folglich geeignete Speichermedien, wie z. B. ein EEPROM, vorgesehen sein, in welchem die Abgleichdaten für die spezielle Meßzelle hinterlegt sind, wobei die Gesamtanordnung schon werkseitig abgeglichen werden kann. Der Anwender kann folglich die gesamte Einheit in sein Meßsystem einbauen und ohne jeglichen Abgleich in Betrieb nehmen.

Nach einem weiteren in Anspruch 5 angegebenen Vorschlag bildet die Druckplatte zusammen mit einem Kammerdeckel eine gasdichte Kammer mit Eintritts- und Austrittsstutzen. Über diese Eintritts- und Austrittsstutzen können benachbarte und aneinandergereihte Meßzellen gemäß Anspruch 6 mittels Kupplungen miteinander verbunden werden, so daß der Anwender ein komplettes Meßsystem in Modulbauweise mit wenigen Handgriffen zusammenstellen kann.

Die erfindungsgemäße Anordnung bietet ferner gemäß Anspruch 7 die Möglichkeit, zwischen Meßzelle und Grundplatte bzw. der erwähnten Gaskammer ein Filter einzusetzen, das mit gleichen Halte- und Justiermitteln ausgestattet ist wie die Meßzelle selbst. Mittels solcher Filter können beispielsweise Querempfindlichkeiten der jeweiligen Meßzellen unterdrückt werden. Montage und Demontage dieses Filters erfolgt in gleicher einfacher Weise ohne Verwendung von Werkzeugen.

Der Gesamtaufbau der Meßanordnung ist derart, daß, bedingt durch die auf die Meßzelle wirkende Federkraft, ein hohes Maß an Stoß- und Vibrationsunempfindlichkeit erreicht wird, das vor allem für tragbare Meßgeräte von Vorteil ist. Ebenso garantiert dieser Aufbau eine lageunabhängige Funktionsweise der Meßzelle.

Die Erfindung ist nachstehend anhand eines bevorzugten Ausführungsbeispieles, das in den Zeichnungen dargestellt ist, im einzelnen erläutert. In den Zeichnungen zeigen:

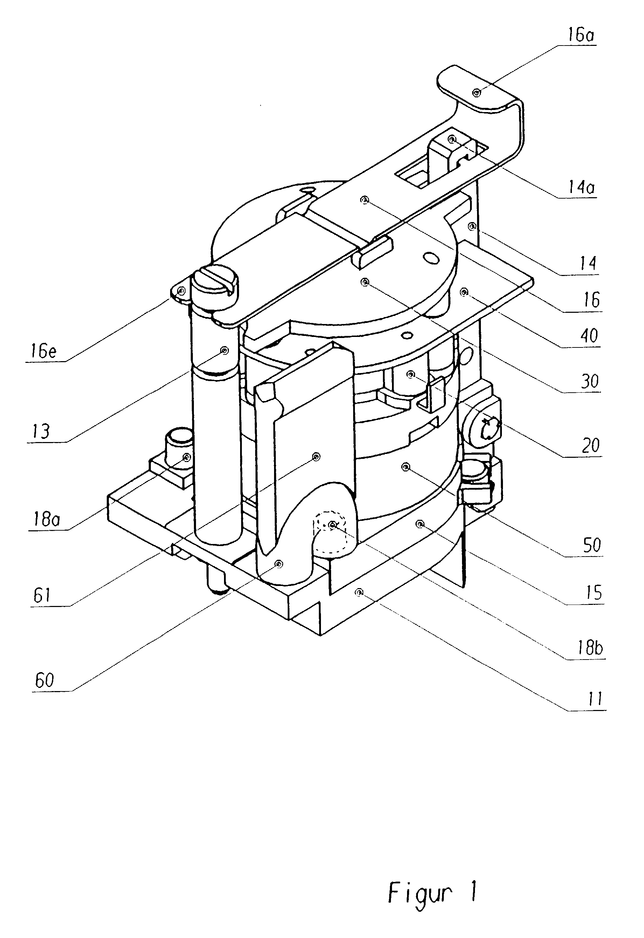

- Figur 1: perspektivische Darstellung der Meßanordnung,

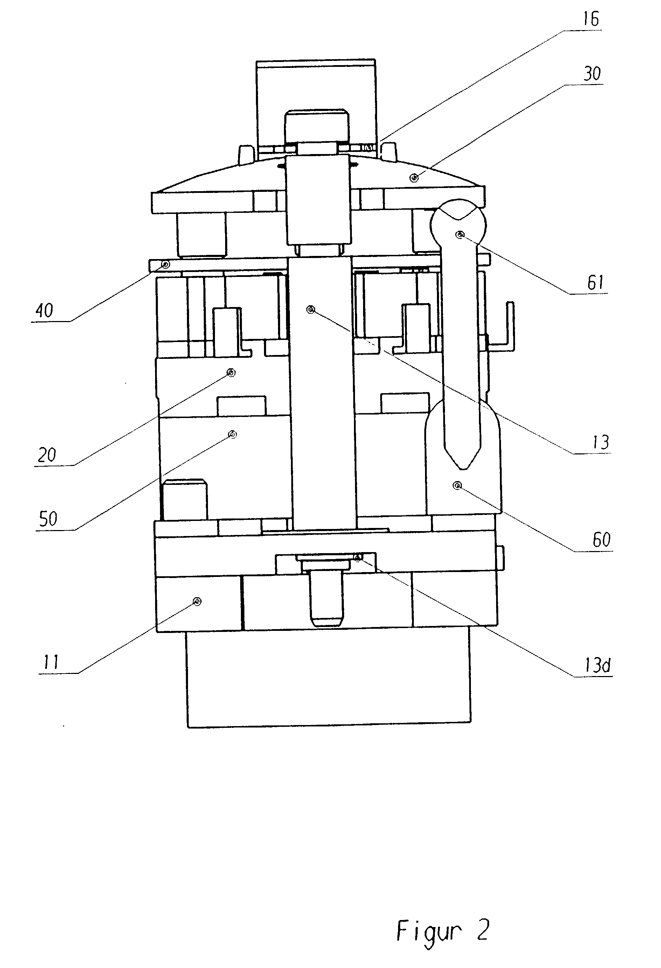

- Figur 2: Seitenansicht der Anordnung gemäß Figur 1 von links gesehen,

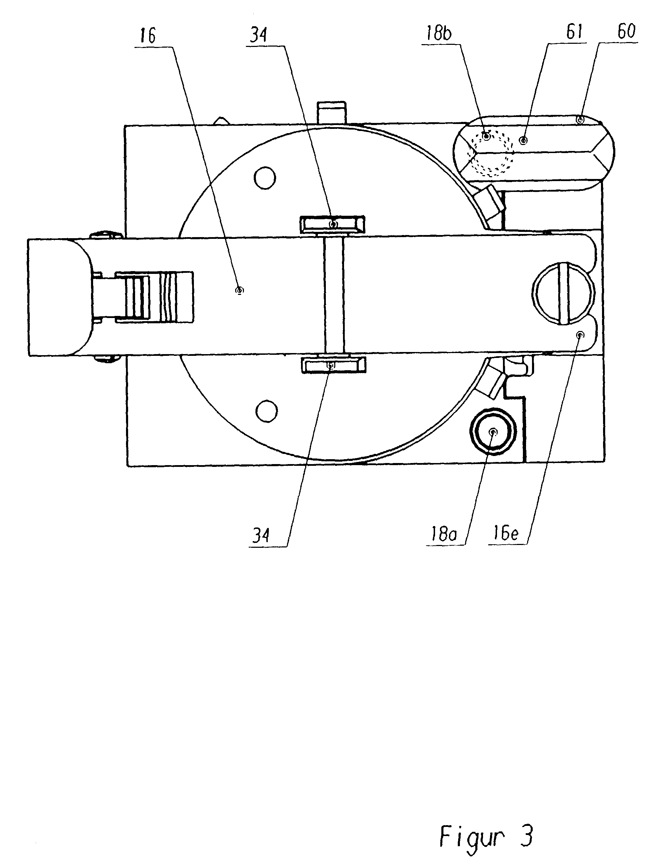

- Figur 3: Aufsicht der Anordnung gemäß Figur 1 und 2 und

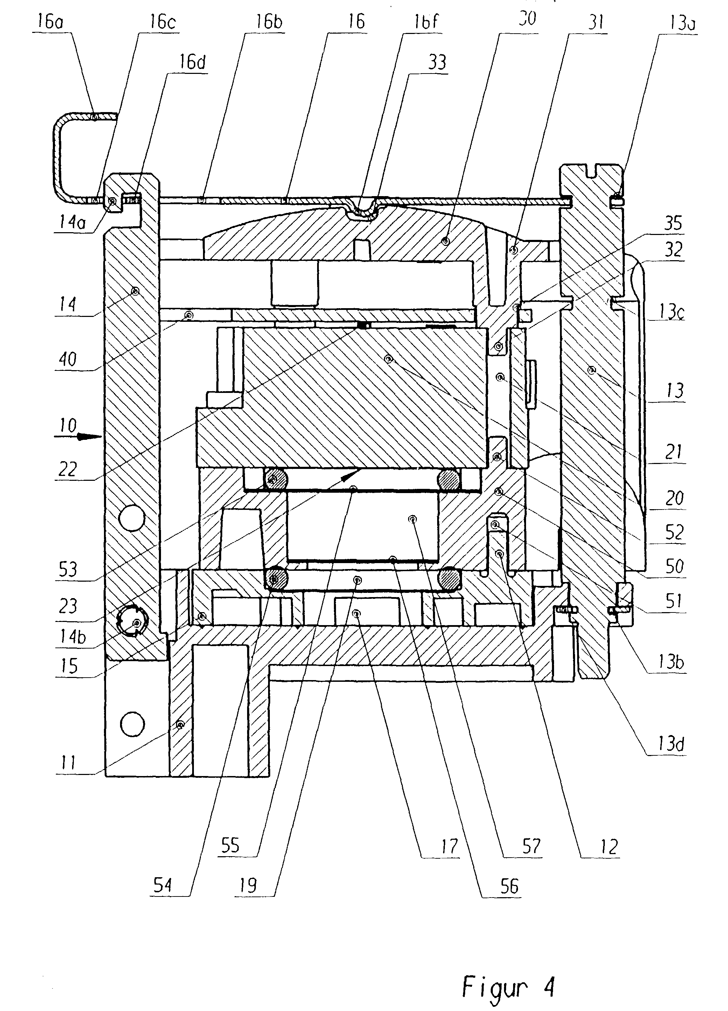

- Figur 4: Axialschnitt der Anordnung.

Die dargestellte Meßanordnung besteht im wesentlichen aus einer Aufnahme 10 mit Grundplatte 11, Stehbolzen 13, Verbindungsarm 14, Druckfeder 16 und Druckplatte 30. Zwischen diesen Elementen sind die Meßzelle 20 mit auf dieser montierter Platine 40 und das Filter 50 eingespannt. Mit der Grundplatte 11 verbunden ist ein Kammerdeckel 15, welcher mit letzterer eine Gaskammer 17 begrenzt.

Der Stehbolzen 13 ist mit seinem unteren Ende in eine Bohrung der Grundplatte 11 eingesetzt und an dieser mit einem in eine Ringnut 13b eingesetzten Sprengring 13d festgelegt. An seinem oberen Ende weist er eine weitere Ringnut 13a auf, in welche die Blattfeder 16 mit ihrem gabelförmigen Ende 16e lösbar eingesetzt ist.

Dem Stehbolzen 13 gegenüberliegend ist der Verbindungsarm 14 angeordnet, welcher über einen Gelenkbolzen 14b mit der Grundplatte 11 schwenkbar verbunden ist. Das obere freie Ende dieses Verbindungsarmes 14 ist als Rasthaken 14a ausgebildet, welcher in Durchbrüche 16b und 16c der Druckfeder 16 eingreift und hierbei im geschlossenen Zustand einen Steg 16d umgreift. Das diesem Rasthaken 14a benachbarte Ende der Druckfeder 16 ist zur Bildung einer die Handhabe erleichternden Betätigungsnase 16a U-förmig umgebogen. Durch Druck auf die Betätigungsnase 16a nach unten läßt sich der Rasthaken 14a lösen. Die Druckfeder 16 weist in ihrem mittleren Bereich eine nach unten gerichtete Abkröpfung 16f auf, welche in eine Rastaufnahme 33 der Druckplatte 30 kraftund formschlüssig eingreift. Zur seitlichen Lagesicherung der Druckfeder 16 sind an der Druckplatte 30 parallel zur Druckfeder 16 verlaufende Führungsstege 34 vorgesehen.

Die konstruktive Gestaltung der Sensoraufnahme 10 gewährleistet auch nach einem Meßzellenwechsel gleiche und reproduzierbare Einspann- und damit Belastungsverhältnisse.

Die Meßzelle 20 selbst ist unmittelbar mit der die Signalaufbereitungsschaltung tragenden Platine 40 steckbar verbunden und zwar über mehrere Kontaktstifte 22, welche in in der Platine 40 vorhandene Kontaktbuchsen eingreifen und damit sowohl der mechanischen als auch elektrischen Verbindung dienen. Die Platine 40 ist hierbei mittels der Ringbünde 35 an den Stützen 31 axial ohne Druckbeanspruchung lagegesichert.

Zur unmittelbaren Übertragung des Einspanndruckes von der Druckplatte 30 auf die Meßzelle 20 ist die Platine 40 mit Durchbrüchen versehen, durch welche Druckstützen 31 ohne Kraftschluß hindurchgreifen. Die Druckstützen 31 liegen mit ihren Stirnflächen auf der Oberseite der Meßzelle 30 auf und greifen zur Zentrierung und Lagejustierung mit Nasen 32 in achsparallel verlaufende Kanäle 21 der Meßzelle 20 ein.

Bei dem dargestellten Ausführungsbeispiel ist zwischen der Grundplatte 11 und der Meßzelle 20 ein Filter 50 geschaltet, während die das nicht dargestellte Filtermaterial beinhaltende Kammer 57 beidseitig durch gasdurchlässige Filtermembranen 55 und 56 begrenzt ist. Die Filterkammer 57 ist so angeordnet, daß sie mit der Gaseintrittsöffnung 23 der Meßzelle kommunizieren kann. Das Filter 50 ist auf seiner Oberseite mit nach oben gerichteten Führungsstiften 52 versehen, die ähnlich ausgebildet und angeordnet sind wie die Nasen 32 der Druckplatte 30. Hierdurch ist gleichfalls eine reproduzierbare Lagesicherung und Ausrichtung des Filters 50 in bezug auf die Meßzelle 20 gewährleistet.

Zur weiteren Ausrichtung ist auch das Filter 50 mit sacklochartigen Aufnahmen 51 versehen, die in gleicher Weise orientiert sind wie die Kanäle 21 der Meßzelle 20 und in welche der Grundplatte 11 zugeordnete Führungsstifte 12 eingreifen. Diese Führungsstifte 12 sind bei dem dargestellten Ausführungsbeispiel Teil des Kammerdeckels 15, der zur Bildung einer Gaskammer 17 auf die Grundplatte 11 aufgesetzt ist.

Auch die Gaskammer 17 ist mit einer der Filterkammer 57 zugeordneten Zentralöffnung 19 versehen. Zwischen Meßzelle 20 einerseits und Kammerdeckel 15 andererseits eingelegte Dichtungsringe, vorzugsweise O-Ringe 53 und 54, gewährleisten einen gasdichten Abschluß des Systems.

Die Gas-Zu- und -Ableitung zur Kammer 17 erfolgt über nach oben offene Eintritts- und Austrittsstutzen 18a und 18b, auf welche Kupplungen 60 aufgesetzt werden können. Diese an ihrer Oberseite mit einem Griffstück 61 ausgestatteten Kupplungen 60 können um die Achse des Eintritts- bzw. Austrittsstutzens 18a bzw. 18b verdreht werden, so daß mit ihnen aneinandergereihte Aufnahmen 10 miteinander verbunden werden können. Auf diese Weise kann mit einer Reihe erfindungsgemäßer Meßanordnungen ein vollkommen offenes und erweiterbares Meßsystem aufgebaut werden.

Mit der oben erläuterten erfindungsgemäßen Meßanordnung werden folgende Vorteile erzielt.
1. Austausch der Meßzelle 20 und der Platine 40 ohne Verwendung von Spezialwerkzeugen.
2. Gleichmäßige Druckbeanspruchung der Meßzelle und drucklos, aber lagesicher an der Meßzelle angebrachte Schaltungsplatine, so daß im Falle des Austausches bereits werkseitig abgeglichene Meßzellen-Platinen-Einheiten eingesetzt und ohne weiteren Abgleich in Betrieb genommen werden können.
3. Keine Gefahr von Signalverfälschungen infolge von mechanischen Spannungen nach erfolgtem Einbau.
4. Wegen der vorgesehenen Modulbauweise ist es möglich, Meßzellen sowie Filter unterschiedlicher Art einzusetzen, soweit die durch die Aufnahme vorgegebenen Abmessungen eingehalten werden.
5. Mehrere Meßanordnungen können zu einem Register mit unterschiedlichen Meßzellen zusammengesetzt werden.
6. Die in der Sensoraufnahme fixierten Meßzellen sind lageunabhängig zu montieren und zu betreiben.

### BEZUGSZEICHENLISTE

- 10: Sensoraufnahme
- 11: Grundplatte
- 12: Führungsstift
- 13: Stehbolzen
- 13a,b,c: Ringnuten
- 13d: Sprengring
- 14: Verbindungsarm
- 14a: Rasthaken
- 14b: Gelenkbolzen
- 15: Kammerdeckel
- 16: Druckfeder
- 16a: Betätigungsnase
- 16b,c: Durchbrüche
- 16d: Steg
- 16e: gabelförmiges Ende, Gabel
- 16f: Abkröpfung
- 17: Kammer
- 18a,b: Eintritts- und Austrittsstutzen
- 19: Zentralöffnung

- 20: Meßzelle (Sensor)
- 21: Kanäle
- 22: Kontaktstift
- 23: Gaseintrittsöffnung

- 30: Druckplatte
- 31: Druckstützen
- 32: Nase
- 33: Rastaufnahme
- 34: Führungsstege
- 35: Ringbund
- 40: Platine

- 50: Filter
- 51: Aufnahme
- 52: Führungsstifte
- 53, 54: Dichtungsringe (O-Ringe)
- 55, 56: Filtermembran
- 57: Kammer

- 60: Kupplung
- 61: Griffstück

## Patentansprüche

1. Meßanordnung zur Untersuchung gasförmiger Medien, im wesentlichen bestehend aus folgenden Bauteilen:
1.1 Eine Meßzelle (20), welche elektrische Signale entsprechend der Konzentration und/oder Zusammensetzung des in diese über eine Öffnung (23) eingeleiteten Gases erzeugt.
1.2 Eine auf einer Platine (40) angeordnete Schaltung zur Auswertung der elektrischen Signale.
1.3 Eine Aufnahme (10) mit einer Grundplatte (11) und einer Deckplatte (30), zwischen welchen die Meßzelle (20) angeordnet ist und welche miteinander verbunden sind,
gekennzeichnet durch folgende Merkmale:
1.4 Die Platine (40) ist unmittelbar an der Meßzelle (20) angebracht und mit dieser elektrisch und mechanisch lösbar verbunden.
1.5 Die Meßzelle (20) mit der Platine (40) ist zwischen derGrund-undderDeckplatte(11, 30) lös- und austauschbar eingespannt.
1.6 Die Deckplatte ist als Druckplatte (30) ausgebildet, welche die Meßzelle (20) gegen die Grundplatte (11) bei gleichmäßiger Druckverteilung preßt.
1.7 Auf die Oberseite der Druckplatte (30) wirkt eine mit der Aufnahme (10) lösbar verbundene Druckfeder (16) mit definierter Spannung.

2. Meßanordnung nach Anspruch 1, gekennzeichnet durch folgende weitere Merkmale:
2.1 Ein Verbindungsarm ist als Stehbolzen (13) ausgebildet, der an einem Ende starr mit der Grundplatte (11) verbunden ist und am anderen Ende eine Ringnut (13a) aufweist.
2.2 Der andere Verbindungsarm (14) ist schwenkbar mit der Grundplatte (11) verbunden und weist an seinem freien Ende einen Rasthaken (14a) auf.
2.3 Die Druckfeder ist als Blattfeder (16) ausgebildet, welche vorzugsweise mit einer Abkröpfung (16f) kraftschlüssig auf der Oberseite der Druckplatte (30) aufliegt.
2.4 Die Druckfeder (60) ist an einem als Gabel (16e) ausgebildeten Ende in die Ringnut (13a) des Stehbolzens (13) lösbar eingesetzt und weist an ihrem anderen Ende eine Aufnahme (16a,b,c) auf, in welche der Rasthaken (14a) des anderen Verbindungsarmes (14) lösbar eingreift.

3. Meßanordnung nach Anspruch 2, dadurch gekennzeichnet, daß die Druckplatte (30) auf ihrer Oberseite eine Rastaufnahme (33), in welche die Blattfeder (16) mit ihrer Abkröpfung (16f) eingreift, sowie beidseitig der Blattfeder (16) in Nachbarschaft der Rastaufnahme gelegene Stege (17d) aufweist.

4. Meßanordnung nach Anspruch 2 oder 3, gekennzeichnet durch folgende weitere Merkmale:
4.1 Die Druckplatte (30) besitzt einen Druckring bzw. Druckstützen (31), welche auf der Oberseite der Meßzelle (20) aufliegen.
4.2 Die Platine (40) ist auf der der Druckplatte (30) zugewandten Oberseite der Meßzelle (20) vorgesehen und weist den Druckstützen (31) entsprechende Ausnehmungen auf.
4.3 Vorzugsweise besitzt die Meßzelle (20) axial verlaufende Kanäle (21) oder dergleichen Aufnahmen, in welche die Druckstützen (31) mit gegenüber diesen im Durchmesser abgesetzten Nasen (32) formschlüssig eingreifen.

5. Meßanordnung nach einem der Ansprüche 1 bis 4, gekennzeichnet durch folgende weitere Merkmale:
5.1 Der Unterseite der Meßzelle (20) gegenüberliegend ist auf die Druckplatte (30) ein Kammerdeckel (15) aufgesetzt, welcher mit dieser eine gasdichte Kammer (17) bildet.
5.2 Die Kammer (17) weist einen Eintritts- und einen Austrittsstutzen (18a,b) auf.
5.3 Die Kammer (17) weist eine Zentralöffnung (19) auf, welche mit der Gaseintrittsöffnung (23) der Meßzelle (20) kommuniziert.

6. Meßanordnung nach Anspruch 5, dadurch gekennzeichnet, daß auf die Eintritts- und/oder Austrittsstutzen (18a,b) eine Kupplung (60) zur gasmäßigen Verbindung benachbarter, aneinanderreihbarer Meßzellen (20) aufsetzbar sind.

7. Meßanordnung nach einem der Ansprüche 1 bis 6, dadurch gekennzeichnet, daß zwischen die Meßzelle (20) und die Grundplatte (11) ein Filter (50) einsetzbar ist, welches den Kanälen (21) der Meßzelle (20) entsprechende Aufnahmen (51) und den Führungsstiften (12) des Kammerdeckels (15) entsprechende Führungsstifte (52) sowie eine zwischen der Zentralöffnung (19) der Kammer (17) und der Gaseintrittsöffnung (23) der Meßzelle (20) gelegene Kammer (57) zur Aufnahme eines Filters besitzt.

8. Meßanordnung nach Anspruch 5 oder 7, dadurch gekennzeichnet, daß die Oberseite des Kammerdeckels (15) axial verlaufende Führungsstifte (12) aufweist, welche in die Kanäle (21) der Meßzelle (20) bzw. die Aufnahmen (51) des Filters (50) eingreifen.

## Claims

1. Measuring arrangement for examining gaseous media, essentially comprising the following components:
1.1 a measuring cell (20) which generates electrical signals corresponding to the concentration and/or composition of the gas fed into it via an opening (23),
1.2 a circuit disposed on a printed circuit board (40) for evaluating the electrical signals,
1.3 a mounting (10) having a base plate (11) and a cover plate (30) between which the measuring cell (20) is disposed and which are joined to one another,
characterized by the following features:
1.4 the printed circuit board (40) is mounted directly on the measuring cell (20) and is detachably connected to it electrically and mechanically,
1.5 the measuring cell (20) with the printed circuit board (40) is detachably and interchangeably clamped between the base plate and the cover plate (11, 30),
1.6 the cover plate is designed as a pressure plate (30) which presses the measuring cell (20) against the base plate (11) with uniform pressure distribution,
1.7 a compression spring (16) detachably joined to the mounting (10) acts on the upper side of the pressure plate (30) with a defined stress.

2. Measuring arrangement according to Claim 1, characterized by the following further features:
2.1 a connecting arm is designed as spacer bolt (13) which is rigidly joined at one end to the base plate (11) and has an annular groove (13a) at the other end,
2.2 the other connecting arm (14) is pivotably joined to the base plate (11) and has a latching hook (14a) at its unsupported end,
2.3 the compression spring is designed as leaf spring (16) which preferably rests on the upper side of the pressure plate (30) in a force-locked manner by means of a crimp (16f),
2.4 the pressure spring (16) is detachably inserted at an end designed as fork (16e) into the annular groove (13a) of the spacer bolt (13) and has, at its other end, a mounting (16a, b, c) in which the latching hook (14a) of the other connecting arm (14) detachably engages.

3. Measuring arrangement according to Claim 2, characterized in that, on its upper side, the pressure plate (30) has a latching mounting (33) in which the leaf spring (16) engages by means of its crimp (16f) and also ridges (34) situated in the vicinity of the latching mounting on both sides of the leaf spring (16).

4. Measuring arrangement according to Claim 2 or 3, characterized by the following further features:
4.1 the pressure plate (30) has a pressure ring or pressure pillars (31) which rest on the upper side of the measuring cell (20),
4.2 the printed circuit board (40) is provided on the upper side of the measuring cell (20), which upper side is adjacent to the pressure plate (30) and has recesses matching the pressure pillars (31),
4.3 the measuring cell (20) preferably has axially extending ducts (21) or similar mountings in which the pressure pillars (31) engage in a shape-locked manner by means of protuberances (32) which are reduced in diameter with respect to said pressure pillars.

5. Measuring arrangement according to one of Claims 1 to 4, characterized by the following further features:
5.1 mounted on the base plate (11) oppcsite the lower side of the measuring cell (20) is a chamber lid (15) which forms with said base plate a gastight chamber (17),
5.2 the chamber (17) has inlet and outlet connecting pipes (18a, b),
5.3 the chamber (17) has a central opening (19) which communicates with the gas inlet opening (23) of the measuring cell (20).

6. Measuring arrangement according to Claim 5, characterized in that a coupling (60) for the gas connection of adjacent, strung-together measuring cells (20) can be mounted on the inlet and/or outlet connecting pipes (18a, b).

7. Measuring arrangement according to one of Claims 1 to 6, characterized in that there can be inserted between the measuring cell (20) and the base plate (11) a filter (50) which has mountings (51) matching the ducts (21) of the measuring cell (20) and guide pins matching the guide pins (52) matching the guide pins (12) of the chamber lid (15) and also a chamber (57), situated between the central opening (19) of the chamber (17) and the gas inlet opening (23) of the measuring cell (20), for receiving a filter.

8. Measuring arrangement according to Claim 5 or 7, characterized in that the upper side of the chamber lid (15) has axially extending guide pins (12) which engage in the ducts (21) of the measuring cell (20) or the mountings (51) of the filter (50).

## Revendications

1. Dispositif de mesure pour l'examen de milieux gazeux, essentiellement composé des éléments suivants :
1.1 Cellule de mesure (20), qui produit des signaux électriques correspondant à la concentration et/ou à la composition du gaz introduit dans la cellule par une ouverture (23).
1.2 Un circuit disposé sur une platine (40) pour évaluer les signaux électriques.
1.3 Un logement (10) avec une plaque de base (11) et une plaque de recouvrement (30), entre lesquelles est disposée la cellule de mesure (20) et qui sont reliées ensemble,
caractérisé par les caractéristiques suivantes :
1.4 La platine (40) est directement appliquée sur la cellule de mesure (20) et est reliée à celle-ci électriquement et mécaniquement de manière détachable,
1.5 La cellule de mesure (20) avec la platine (40) est montée détachable et échangeable entre la plaque de base et la plaque de recouvrement (11, 30).
1.6 La plaque de recouvrement est réalisée comme une plaque de pression (30), qui presse la cellule de mesure (20) contre la plaque de base (11) avec une répartition homogène de pression.
1.7 Sur la face supérieure de la plaque de pression (30) agit un ressort de pression relié mais détachable avec le logement (10) avec une tension définie.

2. Dispositif de mesure selon la revendication 1,
caractérisé par les caractéristiques suivantes :
2.1 Un bras de liaison est réalisé comme un goujon fileté (13), qui est relié à une extrémité rigidement à la plaque de base (11) et comporte à l'autre extrémité une rainure annulaire (13a).
2.2 L'autre bras de liaison (14) est relié pivotable à la plaque de base (11) et comporte à son extrémité libre un crochet d'arrêt (14a).
2.3 Le ressort de pression est un ressort à lames (16), qui appuie de préférence avec un coude (16f) en liaison de force sur la face supérieure de la plaque de pression (30).
2.4 Le ressort de pression (60) est inséré mais détachable à une extrémité réalisée en fourche (16e) dans la rainure annulaire (13a) du goujon fileté (13) et il comporte à son autre extrémité un logement (16a,b,c), dans lequel le crochet d'arrêt (14a) de l'autre bras de liaison (14) s'encliquette de manière détachable.

3. Dispositif de mesure selon la revendication 2,
caractérisé en ce que
la plaque de pression (30) comporte sur sa face supérieure un logement d'arrêt (33) dans lequel s'insère le ressort à lames (16) avec son coude (16f) ainsi que de chaque côté du ressort à lames (16) des entretoises (17d) placées au voisinage du logement d'arrêt;

4. Dispositif de mesure selon les revendications 2 ou 3,
caractérisé par les autres caractéristiques suivantes :
4.1 La plaque de pression (30) possède un anneau de pression ou tubulure de pression (31), qui appuie sur la face supérieure de la cellule de mesure (20).
4.2 La platine (40) est prévue sur la face supérieure de la cellule de mesure (20) tournée vers la plaque de pression (30) et comporte les évidements correspondant à la tubulure de pression (31).
4.3 La cellule de mesure (20) possède de préférence des canaux (21) ou logements équivalents se développant axialement dans lesquels les tubulures de pression (31) pénètrent en liaison de forme avec des nez (32) décalés en diamètre par rapport aux canaux.

5. Dispositif de mesure selon une des revendications 1 à 4,
caractérisé par les autres caractéristiques suivantes :
5.1 Un couvercle de chambre (15) est appliqué à la face inférieure opposée à la cellule de mesure (20) contre la plaque de pression (30), couvercle qui forme avec la cellule une chambre (17) étanche aux gaz.
5.2 La chambre (17) comporte un ajutage d'entrée et un ajutage de sortie (18a,b).
5.3 La chambre (17) comporte une ouverture centrale (19), qui communique avec l'ouverture d'entrée de gaz (23) de la cellule de mesure (20).

6. Dispositif de mesure selon la revendication 5,
caractérisé en ce que
sur l'ajutage d'entrée et/ou l'ajutage de sortie (18a, 18b) sont applicables un accouplement (60) pour la liaison gazeuse de cellules de mesure (20) voisines pouvant être alignées.

7. Dispositif de mesure selon une des revendications 1 à 6,
caractérisé en ce qu'
entre la cellule de mesure (20) et la plaque de base (11) on peut insérer un filtre (50) qui possède des logements (51) correspondant aux canaux (21) de la cellule de mesure (20) et des broches de guidage (52) correspondant aux broches de guidage (12) du couvercle de chambre (15) ainsi qu'une chambre (57) destinée à recevoir un filtre placée entre l'ouverture centrale (19) de la chambre (17) et l'ouverture d'entrée de gaz (23) de la cellule de mesure (20).

8. Dispositif de mesure selon les revendications 5 ou 7,
caractérisé en ce que
la face supérieure du couvercle de chambre (15) comporte des broches de guidage (12) se développant axialement qui s'enclenchent dans les canaux (21) de la cellule de mesure (20) ou dans les logements (51) du filtre (50).
